# EUROPEAN PATENT APPLICATION

(11) **EP 4 806 433 A1**
(43) Date of publication of application: **16.09.2026**
(21) Application number: 25163738.5
(22) Date of filing: 14.03.2025
(51) Int. Cl.: A61K 31/225, A61K 39/00, A61K 40/11, A61K 45/06, A61P 29/00, A61P 37/00, A61P 37/02

(54) **COMBINATION OF IMMUNOMODULATORY AGENT AND ACTIVATED TREG FOR TREATING AUTOIMMUNE AND AUTOINFLAMMATORY DISEASES**

(71) Applicant: ActiTrexx GmbH, 55131 Mainz (DE)
(72) Inventor: Schneider, Franz-Joseph, 1120 Wien (AT); Jonuleit, Helmut, 65428 Rüsselsheim (DE); Schlöder, Janine, 55118 Mainz (DE)
(74) Representative: Patentanwälte Dr. Keller, Schwertfeger Partnerschaft mbB

(57) **Abstract**

The present invention relates to an immunologically effective combination and a pharmaceutical composition, comprising at least one immunomodulatory agent and a regulatory T cell preparation for use in the prevention or treatment of an autoinflammatory or autoimmune disease. The invention also relates to the use of an immunologically effective combination for the manufacturing of a medicament that promotes and restores immune tolerance in a human or animal subject.

## Description

The present invention relates to immunologically effective combinations, comprising at least one immunomodulatory agent and a preparation of isolated CD4-stimulated regulatory T cells (activated Treg) as an immunoregulatory agent for use in the prevention or treatment of an autoimmune disease or autoinflammatory disease. Furthermore, the invention relates to pharmaceutical compositions, comprising an immunologically effective combination and the use of such pharmaceutical compositions in the prevention or treatment of an autoimmune disease or autoinflammatory disease.

Autoimmune and autoinflammatory diseases represent a broad and heterogeneous group of disorders characterized by an aberrant immune response in which the body's defense system mistakenly targets its own tissues. This loss of immune tolerance results in chronic inflammation and progressive tissue damage that can affect virtually any organ system. Common autoimmune and autoinflammatory diseases include rheumatoid arthritis, systemic lupus erythematosus, diabetes type 1, multiple sclerosis, inflammatory bowel diseases, ulcerative colitis, psoriasis, and many others. Although the specific pathology varies from joint destruction in rheumatoid arthritis to demyelination in multiple sclerosis, the underlying mechanism generally involves complex interactions between innate and adaptive immune responses.

Central to these conditions is an immune dysregulation and the breakdown of self-tolerance, where autoreactive lymphocytes become activated. This leads to the production of autoantibodies and/or the release of inflammatory cytokines (e.g., TNF-alpha, IL-6, IL-17) that perpetuate inflammation and drive tissue injury. Some autoimmune diseases target specific organs such as the pancreas in diabetes type 1 or the myelin sheath in multiple sclerosis, while others, like systemic lupus erythematosus, involve multiple organs simultaneously.

Although autoimmune and autoinflammatory diseases differ in their primary trigger, local environment and pathologies, all diseases lead to similar immunological cascades with similar signaling pathways and autoreactive lymphocytes. These components get activated and regulated to varying degrees depending on its underlying autoimmune trigger, the tissue localization and inflammatory environment.

The management of autoimmune and autoinflammatory diseases typically involves a combination of symptomatic relief and long-term immunomodulation with small molecules and biologics, called immunosuppressive drugs, disease-modifying drugs, immunomodulating drugs or anti-inflammatory drugs. However, not all patients respond adequately to available treatments, and some may experience relapses or significant side effects.

Natural CD4+CD25+Foxp3+ regulatory T cells (Treg) represent 4-7% of the CD4+ T cell population and are essential for regulating peripheral tolerance and immune homeostasis. Early phase clinical trials have demonstrated proof-of-principle for the use of isolated and expanded polyclonal Treg in the treatment of inflammatory disorders, transplant rejection and autoimmune diseases. Treg exert their suppressive effects via a plethora of mechanisms, acting on various targets. These include modulating the cytokine microenvironment, metabolic disruption of target T cells, regulating the activation capacity of dendritic cells, and direct cytolysis. Treg have been shown to control autoimmune responses, as well as inflammation and tissue destruction, by their ability to suppress the function of many cell types of the immune system. Consequently, the adoptive transfer of Treg is regarded as a promising treatment option for undesired immune reactions such as autoimmunity, graft rejection, or graft-versus-host disease (GvHD). Successful prevention or control of antigen-specific immunity by Treg has been demonstrated in a variety of animal models.

Despite these approaches, clinical data so far show that blocking just one signaling pathway or inflammatory mediator is not enough to effectively control and stop inflammation in autoimmune diseases in all patients. Even combinations of anti-inflammatory drugs, immune modulatory drugs and disease-modifying drugs targeting different signaling pathways show no significant improvement to a monotherapy.

New strategies to attenuate autoimmune-mediated reactivity and inflammation are urgently needed to decrease and switch off the inflammatory response and to restore immune tolerance without comprehensively suppressing general immunity.

Against this background, it is the object of the present invention to provide novel approaches for the prevention or treatment of autoimmune and autoinflammatory diseases that are able to modulate, suppress or modify immune responses in a subject without specifically targeting immune tolerance mechanisms, and to promote and restore immune tolerance by activating Treg and thereby suppressing autoinflammatory T effector cells.

This object is solved by an immunologically effective combination, comprising at least one immunomodulatory agent and a preparation of isolated CD4-activated regulatory T cells (activated Treg) without expansion/proliferation or genetic modulation of isolated cells before transfer.

In the following, a cell preparation of isolated CD4-activated or CD4-stimulated regulatory T cells is abbreviated by the term "activated Treg". Activated Treg are produced by isolating and purifying regulatory T cells from blood products of autologous or allogeneic donors. Activation of regulatory T cells is carried out by using an activator of regulatory T cells such as anti-CD4-specific antibodies or GP120 (HIV-1 glycoprotein gp120). The term "Treg" as used herein designates regulatory CD4+CD25+ T cells that can be activated via binding of a Treg activator to CD4.

The present invention is based on the surprising finding that a combination of an immunomodulatory agent and activated Treg leads to attenuated inflammation, while activated regulatory T cells downregulate an autoimmune or autoinflammatory T cell response. As shown herein, these underlying mechanisms apply to any immunomodulatory agent that modulates, suppresses or modifies immune responses in a subject without specifically targeting immune tolerance. Based on these principles, the inventive immunologically effective combination provides a novel therapeutic approach and is thus suitable in the treatment or prevention of a variety of autoimmune and autoinflammatory diseases, either by administering a therapeutically effective amount or pharmaceutically effective amount of at least one immunomodulatory agent prior to or concurrently with a preparation of isolated and purified population of activated regulatory T cells to a subject in need thereof. The administering may be repeated as necessary or desired to result in a desired level of attenuated inflammation and autoimmune T cell response.

A subject in need thereof refers to any subject or individual who could benefit from the administration of an immunologically effective combination or method of treatment described herein. In certain embodiments, a subject in need thereof is a subject predisposed for the development of an autoimmune or autoinflammatory disease; a subject having one or more disorders related to an autoimmune or autoinflammatory disease but not exhibiting any clinical symptoms; and/or a subject exhibiting symptoms of an autoimmune or autoinflammatory disease. In one aspect "the subject in need thereof" refers to a vertebrate, such as a mammal. Mammals include, but are not limited to, humans, other primates, rodents (i.e., mice, rats, and hamsters), farm animals, sport animals and pets. In one embodiment, the subject is a mammal such as a human. In certain embodiments, the combinations and methods find use in experimental animals, in veterinary application, and/or in the development of animal models for disease.

A "therapeutically effective amount" or "pharmaceutically effective amount" means the amount of an immunologically effective combination that, when administered to a subject for treating an autoimmune or autoinflammatory disease, is sufficient to affect such treatment. Thus a "therapeutically effective amount" is an amount indicated for treatment while not exceeding an amount which may cause significant adverse effects. The "therapeutically effective amount" will vary depending on the specific immunologically effective combination, and will also be determined by physical and physiological factors such the age, body weight, and/or clinical history of the subject to be treated. Methods for evaluating the effectiveness of therapeutic treatments are known to those of skill in the art.

"An immunologically effective combination" means a single composition containing at least one immunomodulatory agent and activated Treg, or two distinct compositions, wherein the first composition contains at least one immunomodulatory agent, and the second composition contains activated Treg. The compositions are configured for administering to a subject in need thereof a therapeutically effective amount or a pharmaceutically effective amount of the at least one immunomodulatory agent prior to or concurrently with activated Treg. The immunologically effective combination can be a combination preparation or combination product of an immunomodulatory agent and activated Treg as immunoregulatory agent.

The immunomodulatory agents as used in the context of the present invention are characterized in that they target signalling pathways in T cells and Treg only. Consequently, autoimmune indications und autoinflammatory diseases can be treated which are either T cell mediated or where T cells are involved in pathogenesis.

All immunomodulatory agents of the present invention have the ability to reduce the activity of pro-inflammatory cytokines, to decrease the activation and proliferation of autoreactive and autoinflammatory immune cells, and to limit the release of mediators that drive inflammation and tissue damage.

The immunomodulatory agents of the present invention contribute to a reduced inflammation, a reduced immune-mediated tissue injury and the modification of the progression of immune-driven diseases. They do not focus on enhancing or restoring regulatory immune tolerance-promoting mechanisms. This is in contrast to immunoregulatory agents that specifically boost regulatory pathways controlled by Treg and allow to restore immune tolerance.

The inventive combination enables to rebalance a dysregulated network of immune and inflammatory signals in autoimmune diseases, thereby resolving autoimmune responses and re-establishing immune-inflammatory homeostasis and immune tolerance.

By using a combination therapy, a broader mechanistic coverage is achieved which benefits treatment. The combination targets biological pathways and mechanisms of disease processes that a single agent does not fully manage. The combined, synergistic effect of the combination of an immunomodulator and activated Treg is greater than the sum of their individual effects. This synergy will improve treatment outcomes, particularly in complex autoimmune diseases where multiple pathways are involved. A monotherapy may only target one specific component of a disease pathway. However, many autoimmune and autoinflammatory diseases involve multiple pathways or redundant systems that can bypass a single blockade. The therapeutic application of the combination according to the present invention can cover a wider range of these processes because distinct drugs are used with different mechanisms and modes of action.

In a first preferred embodiment, the immunomodulatory agent modulates, suppresses or modifies immune responses in a subject without specifically targeting immune tolerance mechanisms.

The invention covers immunomodulatory agents that uses pharmaceutical substances or active pharmaceutical ingredients falling under the International Nonproprietary Names (INN). INN facilitate the identification of pharmaceutical substances or active pharmaceutical ingredients. Each INN is a unique name that is globally recognized and is public property. A nonproprietary name is also known as a generic name. Examples of INN used in the present invention include, but are not limited to Tocilizumab, Sarilumab, (IL6R inhibitors), Ixekizumab, Secukinumab, Bimekizumab, Brodalumab (IL17 inhibitors), Risankizumab, Ustekinumab, Guselkumab (IL-23 inhibitors), Adalimumab, Etanercept, Infliximab (TNF-alpha inhibitors), Nerandomilast, Apremilast, or Roflumilast (PDE4 inhibitors), Rituximab, Ocrelizumab, Ublituximab, Ofatumumab (CD20 inhibitors), Abatacept (CD80/86 inhibitor), Vedolizumab (integrin blocker), Figolimod, Ozanimod, Ponesimod, Siponimod (sphingosine 1-phosphate receptor modulator), Alemtuzumab (lymphocyte-depleting agent), Frexalimab, Dazodalibep (CD40L blocker), Vidofludimus Calcium (pyrimidine synthesis inhibitor), Aldesleukin (IL-2), Belimumab (B cell inhibitor).

In preferred variants, the immunomodulatory agent is selected from the group consisting of IL-6R inhibitors, IL-17 inhibitors, IL-23 inhibitors, TNF-alpha inhibitors, PDE4 inhibitors, interferon, fumarate immunomodulators.

In alternative embodiments, said immunomodulatory agent is selected from the group consisting of methotrexate, retinoids (e.g. Acitretin), IL-2, CD20 inhibitors, CD80/86 inhibitors, hydroxychloroquine, integrin blockers, sphingosine 1-phosphate receptor modulator, lymphocyte-depleting agents, glatiramer acetate, CD40 inhibitors, preferably CD40L blockers, pyrimidine synthesis inhibitors, B cell inhibitors.

In some embodiments, the IL-6R inhibitor is an anti-IL6R antibody.

In some embodiments, the IL-17 inhibitor is an anti-IL17R antibody.

In some embodiments, the IL-23 inhibitor is an anti-IL23R antibody.

In some embodiments, the TNF-alpha inhibitor is an anti-TNF-alpha antibody or Etanercept.

In some embodiments, the PDE4 inhibitor is and PDE4B inhibitor.

In some embodiments, the interferon is interferon-beta-1a or interferon-beta-1b.

In some embodiments, the fumarate immunomodulator is dimethyl fumarate (DMF), diroximel fumarate or monomethyl fumarate.

In further preferred embodiments, the IL-6R inhibitor is Tocilizumab or Sarilumab

In further preferred embodiments, the IL-17 inhibitor is Ixekizumab, Secukinumab, Bimekizumab, or Brodalumab.

In further preferred embodiments, the IL-23 inhibitor is Risankizumab, Ustekinumab, or Guselkumab.

In further preferred embodiments, the TNF-alpha inhibitor is Adalimumab, Etanercept, or Infliximab.

In further preferred embodiments, the PDE4 inhibitor is Nerandomilast, Apremilast, or Roflumilast.

In further preferred embodiments, the interferon is interferon-beta-1a.

In further preferred embodiments, the fumarate immunomodulator is DMF.

In alternative embodiments, the CD20 inhibitor is Rituximab, Ocrelizumab, Ublituximab, or Ofatumumab.

In alternative embodiments, the CD80/86 inhibitor is Abatacept.

In alternative embodiments, the integrin blocker is Vedolizumab.

In alternative embodiments, the sphingosine 1-phosphate receptor modulator is Figolimod, Ozanimod, Ponesimod, or Siponimod.

In alternative embodiments, the lymphocyte-depleting agent is Alemtuzumab.

In alternative embodiments, the CD40L blocker is Frexalimab or Dazodalibep.

In alternative embodiments, the pyrimidine synthesis inhibitor is Vidofludimus Calcium.

In alternative embodiments, the IL-2 is Aldesleukin.

In alternative embodiments, the B cell inhibitor is Belimumab.

Activated Treg of the present invention relate to a population of isolated and purified activated regulatory T cells that are used as an immunoregulatory agent promoting and restoring immune tolerance and thereby suppressing autoinflammatory and autoimmune T effector cells. Regulatory T cells are stimulated by anti-CD4 antibodies or other CD-4 specific agents. In a preferred embodiment, the regulatory T cells are activated by a CD4-specific Treg activator by stimulating Treg in an ex vivo approach, where Treg are activated outside of the human body. In such an ex vivo approach, blood of a donor is taken, and Treg are stimulated by addition of a Treg activator.

In some embodiments, the Treg activator used for activation of regulatory T cells in an ex vivo approach is an anti-CD4 antibody or a functional fragment thereof. In alternative embodiments, the Treg activator is GP120 or a biologically active fragment thereof. A biologically active fragment of GP120 has the ability to bind to its respective CD4 binding site and to promote Treg activation. The invention also comprises polypeptides or proteins bearing the CD4 binding sites of GP120. The invention furthermore comprises variants, mutants and modified versions of GP120. Activation of Treg by a Treg activator can be carried ex vivo. For example, in an ex vivo approach, blood from a donor can be used to isolate and purify regulatory T cells, and subsequent activation is carried out by a Treg activator. In case of an allogeneic donor, the activated Treg can then be used for treatment of another patient that is not the donor. In case of an autologous donor, the activated Treg can be reinfused into the donor.

Functional fragments of anti-CD4 antibodies are a part of an anti-CD4 antibody comprising a heavy chain polypeptide and/or light chain polypeptide having part or all of the binding activity as the antibody from which the functional fragment is derived. The antibody of the present invention preferably comprises both the heavy chain variable region and the light chain variable region. Examples of the functional fragment include Fd, Fv, Fab, F(ab'), F(ab)2, F(ab')2, single chain Fv (scFv), a diabody, a triabody, a tetrabody, and a minibody. Preferably, the antibody fragment is a Fab fragment. The antibodies or fragments thereof have the ability to bind to a CD4 epitope on T cells, wherein binding of the antibody or fragment thereof results in an activation of regulatory T cells. The antibody of the present invention is preferably derived from a vertebrate such as a human, mouse, rat, cow, rabbit, goat, sheep, and guinea pig. In a preferred embodiment, the anti-CD4 antibody is a humanized monoclonal antibody.

In a further preferred embodiment, the anti-CD4 antibody is Tregalizumab or Palixizumab.

The immunologically effective combination of the invention is also suitable to be used in a method of treatment of an autoinflammatory disease. In a first aspect, the at least one immunomodulatory agent is provided in a first dosage form and the activated Treg are provided as a second dosage form, wherein a dosage regimen provides for the first dosage form to be administered to a subject simultaneously with or prior to the second dosage form.

The present invention also comprises the administration of more than one immunomodulatory agent as described herein. Preferably, the autoinflammatory disease is any one of psoriasis, rheumatoid arthritis, Sjögren's syndrome, diabetes type-1, Crohn's disease and ulcerative colitis, multiple sclerosis, psoriatic arthritis, systemic lupus erythematosus (SLE), uveitis, systemic sclerosis, neuro sarcoidosis.

In preferred embodiments, for the treatment of
a. psoriasis, the immunomodulatory agent is any one of TNF-alpha inhibitor, IL-17 inhibitor, PDE4 inhibitor, interferon, or fumarate immunomodulator;
b. multiple sclerosis, the immunomodulatory agent is any one of fumarate immunomodulator, TNF-alpha inhibitor, IL-6R inhibitor, IL-17 inhibitor, PDE4 inhibitor, or interferon;
c. systemic sclerosis, the immunomodulatory agent is any one TNF-alpha inhibitor, IL-6R inhibitor, or PDE4 inhibitor;
d. rheumatoid arthritis, the immunomodulatory agent is any one TNF-alpha inhibitor, IL-6R inhibitor, or PDE4 inhibitor.
e. Sjögren's syndrome, the immunomodulatory agent is a PDE4 inhibitor,
f. diabetes type-1, the immunomodulatory agent is a PDE4 inhibitor,
g. Crohn's disease and ulcerative colitis, the immunomodulatory agent is any one of TNF-alpha inhibitor, or PDE4 inhibitor,
h. psoriatic arthritis, the immunomodulatory agent is any one of IL-17 inhibitor, TNF-alpha inhibitor, or PDE4 inhibitor,
i. systemic lupus erythematosus (SLE), the immunomodulatory agent is a PDE4 inhibitor,
j. uveitis, the immunomodulatory agent is any one of TNF-alpha inhibitor, or PDE4 inhibitor,
k. neuro sarcoidosis, the immunomodulatory agent is a TNF-alpha inhibitor.

In preferred embodiments, selected immunomodulatory agents as summarized in Table 1 are used for the indicated medical indications in combination with any Treg cell activator such as GP120 or anti-CD4 antibody:

**Table 1:**

| Psoriasis | |
|---|---|
| TNF-alpha Inhibitors | Adalimumab (mAb), Etanercept (Fusion protein TNFR-Ig), Infliximab (mAb) |
| IL-23 Inhibitors | Risankizumab (mAb), Ustekinumab (mAb IL23/12), Guselkumab (mAb) |
| IL-17 Inhibitors | Secukinumab (mAb IL17A), Ixekizumab (mAb IL17A), Bimekizumab (mAb IL17A/F), Brodalumab (mAb IL17 RA) |
| PDE4 Inhibitors | Nerandomilast, Apremilast, Roflumilast |
| Interferon | Interferon-beta-1a, Interferon-beta-1b |
| Fumarate Immunomodulators | dimethyl fumarate, diroximel fumarate, monomethyl fumarate |
| Antimetabolite | |
| Immunosuppressants | Methotrexate |
| Retinoids | Acitretin |

| Rheumatoid Arthritis | |
|---|---|
| TNF-alpha Inhibitors | Adalimumab (mAb), Etanercept (Fusion protein TNFR-IgG), Infliximab (mAb) |
| IL-6R Inhibitors | Tocilizumab (mAb, IL6R), Sarilumab (mAb, IL6R) |
| CD20 Inhibitors | Rituximab (mAb), Ocrelizumab (mAb), Ofatumumab (mAb) |
| Antimetabolite | |
| Immunosuppresants | Methotrexate |
| CD80/86 Inhibitors | Abatacept (CTLA-4Ig) |
| PDE4 Inhibitors | Nerandomilast, Apremilast, Roflumilast |

| Sjögren's Syndrome | |
|---|---|
| CD40 Inhibitors | Frexalimab (mAb Anti-CD40L), Dazodalibep (fusion protein) |
| CD20 Inhibitors | Rituximab (mAb), Ocrelizumab (mAb), Ofatumumab (mAb) |
| Antimetabolite | |
| Immunosuppresants | Methotrexate |
| Antimalarials | Hydroxychloroquine |
| PDE4 Inhibitors | Nerandomilast, Apremilast, Roflumilast |

| Diabetes Type-1 | |
|---|---|
| CD80/86 Inhibitors | Abatacept (CTLA-4Ig) |
| CD20 Inhibitors | Rituximab (mAb), Ocrelizumab (mAb), Ofatumumab (mAb) |
| Interleukin-2 | Aldesleukin |
| PDE4 Inhibitors | Nerandomilast, Apremilast, Roflumilast |

| Crohn's Disease and Ulcerative Colitis | |
|---|---|
| TNF-alpha Inhibitors | Adalimumab (mAb), Etanercept (Fusion protein TNFR-Ig), Infliximab (mAb) |
| IL-23 Inhibitors | Risankizumab (mAb), Ustekinumab (mAb IL23/12), Guselkumab (mAb) |
| Integrin Blockers | Vedolizumab (mAb alpha4β7 integrin) |
| Antimetabolite | |
| Immunosuppressants | Methotrexate |
| PDE4 Inhibitors | Nerandomilast, Apremilast, Roflumilast |

| Multiple Sclerosis | |
|---|---|
| IL-17 Inhibitors | Secukinumab (mAb IL17A), Ixekizumab (mAb IL17A), Bimekizumab (mAb IL17A/F), Brodalumab (mAb IL17 RA) |
| Fumarate Immunomodulators | dimethyl fumarate, diroximel fumarate, monomethyl fumarate |
| Sphingosine 1-phosphate Receptor | |
| Modulator | Fingolimod, Ozanimod, Ponesimod, Siponimod small molecules |
| CD20 Inhibitors | Rituximab (mAb), Ocrelizumab (mAb), Ofatumumab (mAb) |
| IL-6R Inhibitors | Tocilizumab (mAb, IL6R), Sarilumab (mAb, IL6R) |
| TNF-alpha Inhibitors | Adalimumab (mAb), Etanercept (fusion protein TNFR-Ig), Infliximab (mAb) |
| PDE4 Inhibitors | Nerandomilast, Apremilast, Roflumilast |
| Lymphocyte-Depleting Biologic | Alemtuzumab (cytolytic mAb, anti-CD52) |
| Interferon | Interferon-beta-1a, Interferon-beta-1b |
| Copolymers Immunomodulators | Glatiramer acetate |
| CD40 Inhibitors | Frexalimab (mAb Anti-CD40L), Dazodalibep (fusion protein) |
| Pyrimidine Synthesis Inhibitors | Vidofludimus Calcium |

| Psoriatic Arthritis | |
|---|---|
| IL-17 Inhibitors | Secukinumab (mAb IL17A), Ixekizumab (mAb IL17A), Bimekizumab (mAb IL17A/F), Brodalumab (mAb IL17 RA) |
| IL-23 Inhibitors | Risankizumab (mAb), Ustekinumab (mAb IL23/12), Guselkumab (mAb) |
| Antimetabolite | |
| Immunosuppressants | Methotrexate |
| TNF-alpha Inhibitors | Adalimumab (mAb), Etanercept (Fusion protein TNFR-IgG), Infliximab (mAb) |
| PDE4 Inhibitors | Nerandomilast, Apremilast, Roflumilast |

| Systemic Lupus Erythematosus | |
|---|---|
| CD20 Inhibitors | Rituximab (mAb), Ocrelizumab (mAb), Ofatumumab (mAb) |
| Antimalarials | Hydroxychloroquine |
| B cell Inhibitors | Belimumab (mAb BAFF/BLyS) |
| IFN Type I Antagonists | Anifrolumab (mAb IFNAR) |
| Complement Inhibitors | Eculizumab (mAb C5) |
| PDE4 Inhibitors | Nerandomilast, Apremilast, Roflumilast |
| Antimetabolite | |
| Immunosuppressants | Methotrexate |

| Uveitis | |
|---|---|
| TNF-alpha Inhibitors | Adalimumab (mAb), Etanercept (Fusion protein TNFR-Ig), Infliximab (mAb), Certolizumab Pegol (mAb-PEG TNFR) |
| Antimetabolite | |
| Immunosuppressants | Methotrexate |
| PDE4 Inhibitors | Nerandomilast, Apremilast, Roflumilast |

| Systemic Sclerosis | |
|---|---|
| Antimetabolite | |
| Immunosuppressants | Methotrexate |
| IL-6R Inhibitors | Tocilizumab (mAb, IL6R), Sarilumab (mAb, IL6R) |
| PDE4 Inhibitors | Nerandomilast, Apremilast, Roflumilast |
| TNF-alpha Inhibitors | Adalimumab (mAb), Etanercept (Fusion protein TNFR-Ig), Infliximab (mAb) |

| Neuro-Sarcoidosis | |
|---|---|
| TNF-alpha Inhibitors Antimalarials | Adalimumab (mAb), Etanercept (Fusion protein TNFR-Ig), Infliximab (mAb) Hydroxychloroquine |

Table 1 summarizes possible selections of immunomodulatory agents together with activated Treg. The invention also comprises the selection of more than one immunomodulatory agent for a combination therapy with activated Treg for the treatment of the indicated autoimmune or autoinflammatory diseases.

For example, for the treatment of psoriasis, the use of any one of TNF-alpha Inhibitors such as Adalimumab (mAb), Etanercept (Fusion protein TNFR-Ig), Infliximab (mAb); IL 23 Inhibitors such as Risankizumab (mAb), Ustekinumab (mAb IL23/12), Guselkumab (mAb); IL 17 Inhibitors such as Secukinumab (mAb IL17A), Ixekizumab (mAb IL17A), Bimekizumab (mAb IL17A/F), Brodalumab (mAb IL17 RA); PDE4 Inhibitors such as Nerandomilast, Apremilast, Roflumilast; Interferon such as Interferon beta 1a, Interferon beta 1b; Fumarate Immunomodulators such as dimethyl fumarate, diroximel fumarate, monomethyl fumarate; Antimetabolite Immunosuppressants such as Methotrexate; Retinoids such as Acitretin in combination with activated Treg is preferred.

As a further example, for the treatment of multiple sclerosis, the use of any one of IL 17 Inhibitors such as Secukinumab (mAb IL17A), Ixekizumab (mAb IL17A), Bimekizumab (mAb IL17A/F), Brodalumab (mAb IL17 RA); Fumarate Immunomodulators such as dimethyl fumarate, diroximel fumarate, monomethyl fumarate; Sphingosine 1-phosphate Receptor Modulator such as Fingolimod, Ozanimod, Ponesimod, Siponimod small molecules; CD20 Inhibitors such as Rituximab (mAb), Ocrelizumab (mAb), Ofatumumab (mAb); IL-6R Inhibitors such as Tocilizumab (mAb, IL6R), Sarilumab (mAb, IL6R); TNF-alpha Inhibitors such as Adalimumab (mAb), Etanercept (fusion protein TNFR-Ig), Infliximab (mAb); PDE4 Inhibitors such as Nerandomilast, Apremilast, Roflumilast; Lymphocyte-Depleting Biologic such as Alemtuzumab (cytolytic mAb, anti-CD52); Interferon such as Interferon beta 1a, Interferon beta 1b; Copolymers Immunomodulators such as Glatiramer acetate; CD40 Inhibitors such as Frexalimab (mAb Anti-CD40L), Dazodalibep (fusion protein); Pyrimidine Synthesis Inhibitors such as Vidofludimus Calcium in combination with activated Treg is preferred.

As a further example, for the treatment of rheumatoid arthritis, the use of any one of TNF-alpha Inhibitors such as Adalimumab (mAb), Etanercept (Fusion protein TNFR-IgG), Infliximab (mAb); IL-6R Inhibitors such as Tocilizumab (mAb, IL6R), Sarilumab (mAb, IL6R); CD20 Inhibitors such as Rituximab (mAb), Ocrelizumab (mAb), Ofatumumab (mAb); Antimetabolite Immunosuppressants such as Methotrexate; CD80/86 Inhibitors such as Abatacept (CTLA-4Ig); PDE4 Inhibitors such as Nerandomilast, Apremilast, Roflumilast in combination with activated Treg is preferred.

As a further example, for the treatment of systemic sclerosis, the use of any one of Antimetabolite Immunosuppressants such as Methotrexate; IL-6R Inhibitors such as Tocilizumab (mAb, IL6R), Sarilumab (mAb, IL6R), PDE4 Inhibitors such as Nerandomilast, Apremilast, Roflumilast; TNF-alpha Inhibitors such as Adalimumab (mAb), Etanercept (Fusion protein TNFR-Ig), Infliximab (mAb) in combination with activated Treg is preferred.

Interestingly, despite the distinct molecular interactions (mechanism of action), the immunomodulatory agents have a common mode of action e.g., ameliorating the cellular activation and/or proliferation of reactive immune cells and reduction of pro-inflammatory cytokines or mechanisms. Since these inflammatory processes are characteristic for autoimmune and autoinflammatory diseases, these agents are suitable for use in various autoimmune or autoinflammatory diseases. This transferability of agents having a common mode of action is also evident when comparing the standard therapies for autoimmune and autoinflammatory diseases. Many agents such as TNF inhibitors are used in the context of different autoimmune or autoinflammatory diseases, including, but limited to psoriasis, rheumatoid arthritis, Crohn's disease and ulcerative colitis multiple sclerosis.

This transferability of agents is also shown by the data presented herein. The panel of agents applied in this patent application have different mechanisms of action, but a common mode of action and subsequently show synergistic efficacy on T cells from healthy donors and autoimmune and autoinflammatory patients. Interestingly, the data show that some agents only become effective in additional diseases through the combination with activated Treg.

The present invention also relates to pharmaceutical compositions, comprising an immunologically effective combination as defined herein and a pharmaceutically acceptable carrier or diluent.

In one aspect, the pharmaceutically composition comprises an immunologically effective combination as defined herein and a pharmaceutically acceptable carrier or diluent for the use in the prevention or treatment of an autoinflammatory disease.

In a preferred embodiment, the immunomodulatory agent and the activated Treg in the immunologically effective combination are provided in a single dose form or in two distinct dose forms. The one embodiment, the single dose form contains both the immunomodulatory agent and the activated Treg. In an alternative embodiment, one dose form contains the immunomodulatory agent and another dose form contains the activated Treg.

The pharmaceutical composition may be formulated with any known pharmaceutically acceptable carrier or diluent as well as any other known adjuvants and excipients in accordance with conventional techniques. The pharmaceutically acceptable carriers, diluents, adjuvants and excipients should be suitable for the chosen inductor of the present invention and the chosen mode of administration. A pharmaceutical composition of the present invention may also include diluents, fillers, salts, buffers, detergents (e. g., a nonionic detergent), stabilizers (e. g., sugars or protein-free amino acids), preservatives, solubilizers, and/or other materials suitable for inclusion in a pharmaceutical composition. In a preferred embodiment, the activated Treg are provided in sterile NaCl + 2% HSA (human serum albumin) in form of an intravenous infusion. In a preferred dosage form, the osmolarity and ion concentrations of the solutions are isotonic to match the milieu of the human body.

The pharmaceutical compositions of the present invention can be formulated by methods known to those skilled in the art. For example, such pharmaceutical compositions can be used parenterally, as injections which are sterile solutions or suspensions including the compositions along with water or another pharmaceutically acceptable liquid. For example, such compositions may be formulated as unit doses that meet the requirements for the preparation of pharmaceuticals by appropriately combining the compositions with pharmaceutically acceptable carriers, diluents, adjuvants or excipients, specifically with sterile water, physiological saline, a vegetable oil, emulsifier, suspension, surfactant, stabilizer, flavoring agent, excipient, vehicle, preservative, binder or such. In such preparations, the amount of active ingredient is adjusted.

The two immunologically active components of the combination of the present invention, i.e. the immunomodulatory agent and the activated Treg can be combined in a single composition for use in the treatment or prevention of an autoinflammatory disease. In alternative embodiments, the immunomodulatory agent and the Activated Treg are provided separately in two distinct compositions.

In a first aspect, the composition containing the immunomodulatory agent and the composition containing the activated Treg can be combined prior to administration to a subject in need thereof. In a second aspect, the composition comprising the immunomodulatory agent and the composition comprising the activated Treg are administered as a single dose or repeated dose to a subject in need thereof. In a third aspect, the composition comprising the immunomodulatory agent and the composition comprising the activated Treg are administered to a subject in need thereof within a desired time interval. For example, the composition comprising the immunomodulatory agent is given to a subject in need thereof at a first time point, while the composition comprising the activated Treg is given to a subject in need thereof at a second time point within a given time interval. It is the aim to have no or only little Treg resistance, so it is important to suppress inflammation sufficiently by the pre-treatment with the immunoregulatory agent before administration of the activated Treg as immunoregulatory agent. Preferably, the time interval for administering a single or repeated dose of the first composition comprising the immunomodulatory agent and administering a single or repeated dose of the second composition comprising the activated Treg is at least 7 days, preferably at least 14 days, more preferably at least 30 days. In more preferred embodiments, the time interval for administering a single or repeated dose of the first composition comprising the immunomodulatory agent and administering a single or repeated dose of the second composition comprising the activated Treg is at least 4 weeks, preferably between 4 weeks and 6 weeks. The administration of the first and/or second composition can be repeated several times. In addition, the number of administrations, the composition and concentration of the compositions comprising the immunomodulatory agent and/or activated Treg can vary. For example, the individual doses given to a subject may vary and depend, inter alia, from gender, age, constitution and state of illness.

In some embodiments, in the pharmaceutical composition of any one of embodiments disclosed herein, the immunomodulatory agent is in the same dosage form as the activated Treg such that the immunomodulatory agent is administered concurrently with the activated Treg. In a preferred embodiment, the dosage form is a single fluid. In some embodiments, the dosage form including the immunomodulatory agent and the activated Treg is prepared by combining a composition including the immunomodulatory agent and a composition including the activated Treg. In some embodiments, in the pharmaceutical composition of any one of embodiments disclosed herein the immunomodulatory agent is in a separate dosage form from the activated Treg such that the immunomodulatory agent can be administered before or after the activated Treg. A delayed administration of the activated Treg relative to the immunomodulatory agent is preferred.

The invention also relates to the use of an immunologically effective combination comprising at least one immunomodulatory agent and activated Treg for the manufacturing of a medicament that promotes and restores immune tolerance in a human or animal subject. The manufacturing process can involve the preparation of a first dosage form, in which the immunomodulatory agent is provided, and a second dosage form, in which the activated Treg are provided, while both dosage forms are prepared to be administered to a subject in need thereof.

In one embodiment, autologous (patient-derived) Treg are isolated from the patient's own blood (peripheral blood mononuclear cells, PBMC).

In another embodiment, allogeneic 3^{rd} party Treg are taken from a healthy donor.

In preferred process, Treg are isolating using anti-CD25 antibodies coupled to magnetic beads. However, also antibodies targeting alternative surface molecules of Treg can be used. In alternative embodiments, fluorescence-activated cell sorting (FACS) or magnetic-activated cell sorting (MACS) are used to isolate and purify Treg. If required, Treg are cultured ex vivo with IL-2 and anti-CD3/CD28 to extend Treg population. In some arrangements, the culturing medium can include rapamycin to favor Treg over conventional T cell expansion. The purity of the isolated Treg can be checked using Treg markers and testing suppressive function. Furthermore, a screening can be conducted to detect contaminations or malfunctions before infusion. Preferably, the medicament containing activated Treg is provided in form that allows intravenous infusion (IV). The clinical response can be monitored, for instance, by analysis of biomarkers, and symptom relief.

In some embodiments, Treg can be delivered directly to the affected tissue to enhance efficacy and reduce systemic immunosuppression by intraarticular, intrathecal or intraperitoneal injection. In some embodiments, activated Treg can be encapsulated in biomaterial scaffolds or hydrogels. In some aspects, microgel or nanogel technology could help sustain Treg release and activity over time.

### Brief Descriptions of the Drawings:

**Figure 1****:** Synergistic effects of activated Treg with different modulators on proliferation of T cells from healthy controls.
**Figure 2****:** Synergistic effects of activated Treg with different modulators on proliferation of T cells from patients with multiple sclerosis.
**Figure 3****:** Synergistic effects of activated Treg with different modulators on proliferation of T cells from patients with psoriasis.
**Figure 4****:** Synergistic effects of activated Treg with different modulators on proliferation of T cells from patients with systemic sclerosis.

### Examples:

The present invention is further illustrated in the following examples. By no means shall the present invention be restricted to these specific examples. Also, the combination of features or even embodiments are encompassed by the spirit of the present invention.

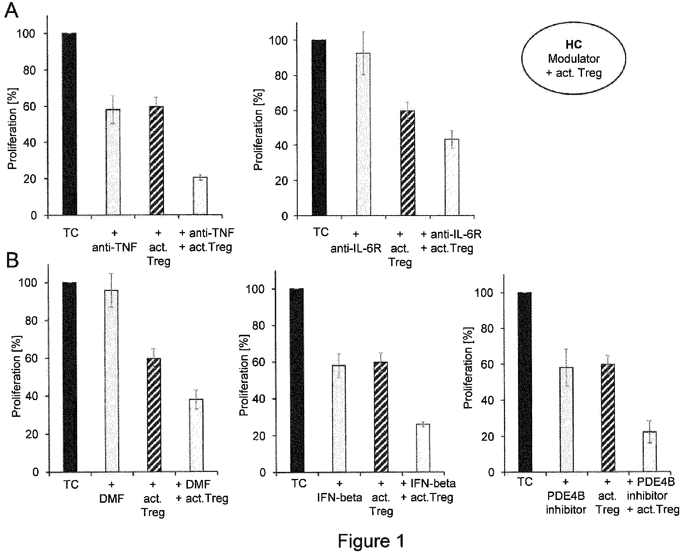

A standard suppression assay was used to determine the T cell modulating activity of immunomodulatory agent alone or in combination with activated Treg. The data of Figures 1 to 4 show the result of a mixed leukocyte reaction (MLR). Isolated T cells from the blood products of healthy donors (HC), patients with multiple sclerosis (MS), psoriasis patients (PSO), and patients with systemic sclerosis (SSC) were stimulated with allogeneic dendritic cells (DC) from independent, healthy donors. The black bars show the optimal proliferation of the alloreactive T cells (from HC and patients), defined as 100% of T cell proliferation. To suppress T cell proliferation, regulatory T cells activated with GP120 (Treg activator) and various immunomodulatory agents (anti-TNF, anti-IL6R, DMF, IFN-beta, PDE4B inhibitor) were used alone or in combination with activated Treg. The influence of these biologicals or combinations of biological and activated Treg is shown as T cell proliferation in percentage (%).

**Figure 1** shows the synergistic effects of activated Treg with different modulators on proliferation of T cells from healthy controls.

T cells from healthy donors (HC) cocultured with allogeneic immature dendritic cells (mixed lymphocyte reaction) in the absence or presence of activated Treg from a third independent healthy donor are shown. Different modulators were added to these cultures. A) From left to right: anti-human TNF-alpha mAb (50 ng/ml), anti-human IL-6R mAb (30 ng/ml). B) From left to right: Dimethyl fumarate (DMF, 2 µM), recombinant human IFN-beta (1 IU/ml), PDE4B inhibitor (2 µM). On day 4 of culture, 37 kBq [³H] Thymidine (³H-Tdr) was added to each well and cells were cultured for an additional 16 h. T cell proliferation was measured by ³H-Tdr incorporation using a liquid beta-scintillation counter. Bars show mean proliferation (in %) ± SEM of n=5 individual experiments.

Figure 1A on the left shows that anti-TNF and activated Treg inhibit T cell proliferation by approximately 40%. In combination (anti-TNF + activated Treg), T cell proliferation is suppressed by up to 80%. Anti-TNF and activated Treg inhibit T cell activation synergistically. Figure 1A on the right shows the result in the assay in presence/absence of anti-IL-6R. In contrast to activated Treg, anti-IL-6R has only a marginal effect on T cell proliferation.

However, activated Treg are able to increase significantly the suppressive effect of anti-IL-6R on T cell proliferation.

Figure 1B on the left shows the suppressive effect of activated Treg in combination with DMF. While DMF alone can hardly influence T cell proliferation, the combination of DMF + activated Treg shows a clear synergistic effect. The proliferation of T cells is suppressed significantly more strongly with this combination of the biological plus activated Treg than with either of these factors alone.

Figure 1B middle shows the effect of IFN-beta and activated Treg on the proliferation of T cells individually and in combination. Both factors suppress T cell proliferation to a comparable extent. In combination (IFN-beta + activated Treg), the synergistic effect is clearly visible. The combination of both components inhibits T cell proliferation significantly stronger than either agent alone.

Figure 1B right shows the influence of PDE4B inhibitors and activated Treg on the proliferation of activated T cells. Individually both factors (PDE4B inhibitor or activated Treg) show a comparable suppressive effect on T cell proliferation (approx. 40%). In combination (PDE4B inhibitor + activated Treg), this suppression of T cell proliferation increases to up to 80%. PDE4B inhibitors and activated Treg synergistically suppress the proliferation of activated T cells.

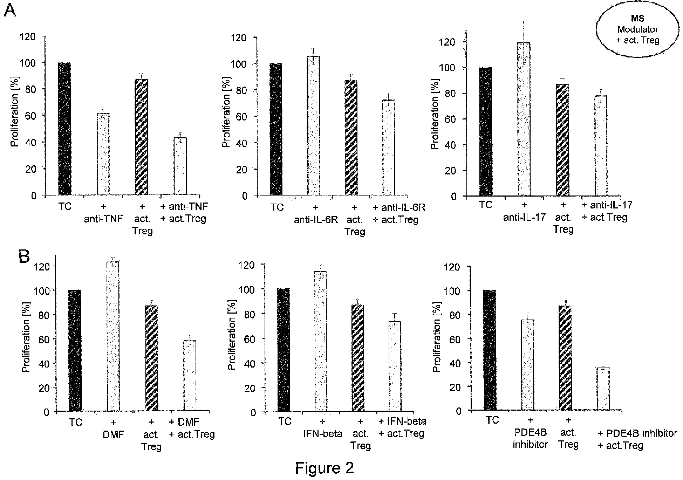

**Figure 2** shows the synergistic effects of activated Treg with different modulators on proliferation of T cells from patients with multiple sclerosis.

T cells from multiple sclerosis patients (MS) were cocultured with allogeneic dendritic cells (mixed lymphocyte reaction) in the absence or presence of activated Treg from a third independent healthy donor (act. Treg). Different modulators were added to these cultures. A) From left to right: anti-human TNF-alpha mAb (50 ng/ml), anti-human IL-6R mAb (30 ng/ml), anti-human IL-17A mAb (50 ng/ml). B) From left to right: Dimethyl fumarate (DMF, 2 µM), recombinant human IFN-beta (1 IU/ml), PDE4B inhibitor (2 µM). On day 4 of culture, 37 kBq [3H] Thymidine (3H-Tdr) was added to each well and cells were cultured for an additional 16 h. T cell proliferation was measured by 3H-Tdr incorporation using a liquid beta-scintillation counter. Bars show mean proliferation (in %) ± SEM of n=3 individual experiments.

Figure 2A left shows the influence of anti-TNF or activated Treg and of a combination of anti-TNF plus activated Treg on the proliferation of T cells isolated from the peripheral blood of MS patients. While activated Treg can only suppress the proliferation of T cells insignificantly due to the known Treg resistance^{3,4}, modulation by anti-TNF leads to a suppression of T cell proliferation of approximately 40%. In any case, the combination of anti-TNF plus activated Treg is more effective than either factor alone. Activated Treg synergistically suppresses the activation of T cells from MS patients with anti-TNF.

Figure 2A middle shows that anti-IL6R is not able to suppress the proliferation of Treg-resistant T cells from the blood of MS patients. In contrast, suppression in combination with activated Treg is clearly detectable. Activated Treg shows synergistic effects with anti-IL6R in the suppression of Treg-resistant T cells from the blood of MS patients.

Figure 2A right shows that anti-IL-17 increases the proliferation of activated T cells from the blood of MS patients (no therapeutic effect), while anti-IL-17 in combination with activated Treg synergistically suppresses the activation of Treg-resistant T cells from MS patients (strong therapeutic effect), even more than is possible with activated Treg alone (intermediate therapeutic effect).

Figure 2B left shows that DMF increases the proliferation of T cells from the blood of MS patients instead of suppressing it. In combination with activated Treg, however, a significant suppression of the previously Treg-resistant T cell proliferation is detectable. The data show that activated Treg synergistically with DMF suppresses the activation of T cells from the blood of MS patients and can significantly increase the therapeutic effect of the drug DMF. Figure 2B middle shows that IFN-beta also promotes the proliferation of Treg-resistant T cells from the blood of MS patients instead of suppressing it. In combination with activated Treg, however, a significant reduction in T cell proliferation is measurable. The data show that activated Treg synergistically inhibits the proliferation of T cells from the blood of MS patients with IFN-beta and can significantly improve the therapeutic success of the biological IFN-beta.

Figure 2B on the right shows that PDE4B inhibitor measurably suppresses the proliferation of T cells from the blood of MS patients, but here too the strongest effect in the combination of activated Treg plus PDE4B inhibitor. Activated Treg shows a synergistic effect with PDE4B inhibitor in the suppression of T cells from the blood of MS patients and can thus significantly increase the therapeutic effect of the drug PDE4B inhibitor.

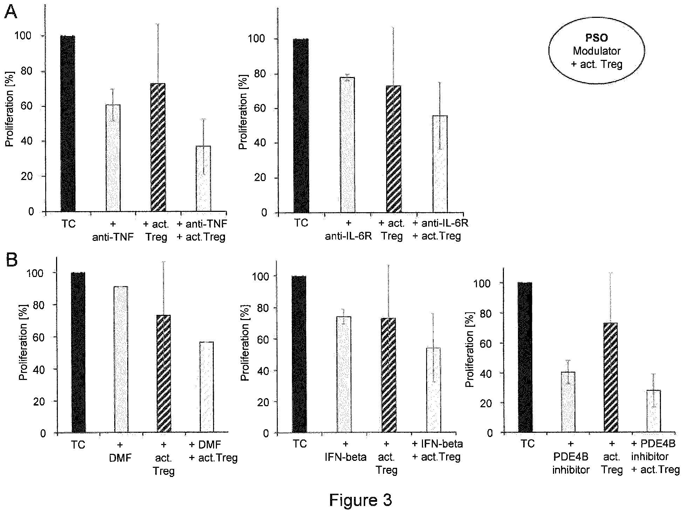

**Figure 3** shows the synergistic effects of activated Treg with different immunomodulatory agents on proliferation of T cells from patients with psoriasis.

T cells from psoriasis patients (PSO) were cocultured with allogeneic dendritic cells (mixed lymphocyte reaction) in the absence or presence of activated Treg from a third independent healthy donor (act. Treg). Different modulators were added to these cultures. A) From left to right: anti-human TNF-alpha mAb (50 ng/ml), anti-human IL-6R mAb (30 ng/ml). B) From left to right: Dimethyl fumarate (DMF, 2 µM), recombinant human IFN-beta (1 IU/ml), PDE4B inhibitor (2 µM).

On day 4 of culture, 37 kBq [3H] Thymidine (3H-Tdr) was added to each well and cells were cultured for an additional 16 h. T cell proliferation was measured by 3H-Tdr incorporation using a liquid beta-scintillation counter. Bars show mean proliferation (in %) ± SEM of n=2 individual experiments.

Figure 3A left shows that anti-TNF suppresses the proliferation of T cells from the peripheral blood of psoriasis patients more than activated Treg. However, the combination of anti-TNF and activated Treg shows the strongest effect. Activated Treg synergistically with anti-TNF suppresses the proliferation of T cells from the blood of psoriasis patients and thus enhances the therapeutic effect of the biologic.

Figure 3A right shows that anti-IL-6R suppresses the proliferation of T cells from the blood of psoriasis patients comparable to activated Treg. However, the combination of anti-IL-6R and activated Treg is significantly more effective. Activated Treg synergistically with anti-IL-6R suppresses the proliferation of T cells from the blood of psoriasis patients, thereby enhancing the therapeutic effect of anti-IL6R.

Figure 3B left shows that DMF alone can only marginally suppress the proliferation of T cells from the blood of psoriasis patients. In combination with activated Treg, the suppression is significant. Activated Treg shows synergistic effects in the suppression of the activation of T cells from psoriasis patients and thus enhances the therapeutic effect of DMF.

Figure 3B middle shows the synergistic effects of IFN-beta and activated Treg on the proliferation of T cells from the blood of psoriasis patients. Both factors alone are less efficient in suppressing T cells than the combination of both. The data show that activated Treg synergistically enhance the therapeutic effect of IFN-beta in suppressing T cells from the blood of psoriasis patients.

Figure 3B right shows that PDE4B inhibitors suppress the proliferation of T cells from the blood of psoriasis patients more than activated Treg. However, the combination of PDE4B inhibitor and activated Treg is again more effective in suppressing T cells than either factor alone. The data show that activated Treg significantly enhances the therapeutic effect of PDE4B inhibitors in suppressing T cell activity in patients with psoriasis.

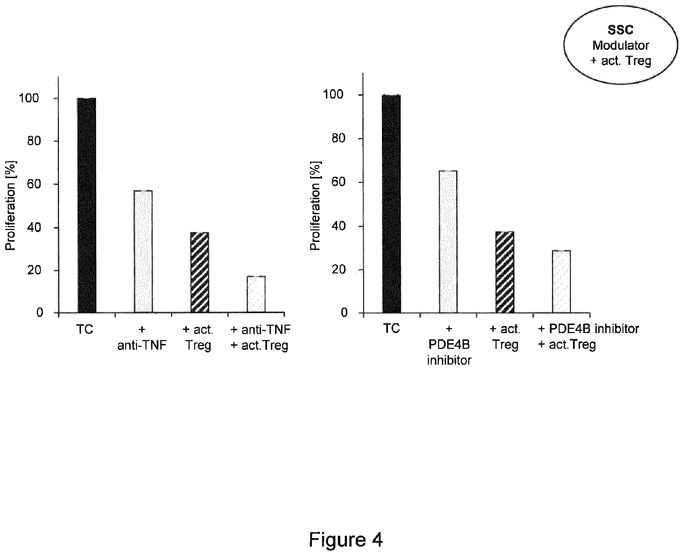

**Figure 4** shows the synergistic effects of activated Treg with different modulators on proliferation of T cells from patients with systemic sclerosis.

T cells from systemic sclerosis patients (SSC) were cocultured with allogeneic dendritic cells (mixed lymphocyte reaction) in the absence or presence of 1 µg/ml GP120. Different modulators were added to these cultures: anti-human TNF-alpha mAb (50 ng/ml), PDE4B inhibitor (2 µM).

On day 4 of culture, 37 kBq [³H] Thymidine (³H-Tdr) was added to each well and cells were cultured for an additional 16 h. T cell proliferation was measured by ³H-Tdr incorporation using a liquid beta-scintillation counter. Bars show proliferation in %.

Figure 4 left shows that activated Treg can suppress the proliferation of T cells from the blood of patients with systemic sclerosis more effectively than anti-TNF. However, this suppression is greatest when activated Treg and anti-TNF are combined. The data show that activated Treg synergistically enhances the suppressive effect of anti-TNF in patients with systemic sclerosis and thus improves the therapeutic effect of anti-TNF as a therapeutic biological.

Figure 4 right shows that activated Treg suppresses the proliferation of T cells from the blood of patients with systemic sclerosis more strongly than a PDE4B inhibitor. However, the combination of activated Treg and PDE4B inhibitor shows the strongest inhibitory effect. Activated Treg shows synergistic effects with PDE4B inhibitors in the suppression of T cells from the blood of patients with systemic sclerosis and thus improves the therapeutic success of the biologic.

In summary, activated Treg can significantly enhance the therapeutic effect (suppression of T cell activation) of different biologics. This applies to biologics that block independent, different signaling pathways and it applies to patients with different autoinflammatory diseases or autoimmune diseases.

The key to the therapeutic-synergistic effect of activated Treg is that activated Treg and the respective biologic suppress signaling pathways in T cells. This means that activated Treg has a synergistic enhancing effect in all autoinflammatory diseases and autoimmune diseases in which unwanted T cell activity is present and in which T cell-modulating biologics are used as therapeutics.

The data also show that a combined application of an immunomodulatory agent and activated Treg is superior to any monotherapy in the treatment of autoimmune disease.

### Materials and Methods

### Isolation and culture of human immune cells

PBMC from either multiple sclerosis (MS) patients, psoriasis (PSO) patients, patients with systemic sclerosis (SSC), or healthy donors (HC) were isolated from peripheral blood within 16 h after blood collection using density gradient centrifugation. Blood was kept at room temperature before PBMC enrichment. After isolation, human cells were cultured in X-VIVO-15 (Lonza, Belgium).

### Generation of dendritic cells

Dendritic cells (DC) were generated from isolated PBMC from the peripheral blood of healthy donors. 10-15x10⁶ PBMC (per well) were seeded in a 6-well culture plate in 2 ml RPMI + 1.5% heat-inactivated blood plasma and incubated for 30 minutes in an incubator at 37 °C and 5% CO₂. During this incubation time, monocytes adhered to the plastic surface. The non-adherent cells were washed off several times with 1 ml pre-warmed PBS and the purity of the monocyte culture was visually checked under a light microscope. Afterwards monocytes were cultured in X-VIVO-15 (3 ml/well) supplemented with 1% heat-inactivated plasma + 400 IU/ml rh GM-CSF (Sargramostim / Leukine) + 200 IU/ml rh IL-4 (Immunotools) for 6 days. On days 2 and 4 of the culture, cells were fed by removing 1 ml of medium per well and replacing it with 1 ml of culture medium supplemented with 800 IU/ml rh GM-CSF. DC were stored frozen in aliquots until use.

### Isolation and CFSE labeling of CD3+ T cells

Untouched CD3+ T cells were isolated using Pan T cell isolation kit (Miltenyi Biotec) according to manufacturer's instructions. The purity of the isolated CD3+ T cells was checked by flow cytometry. For this purpose, cells were stained with the following antibodies: anti-human CD3 FITC (UCHT1), anti-human CD4 PE-Cy7 (RPA-T4), anti-human CD8 APC (RPA-T8), all from BD Pharmingen. Stained cells were measured on LSRII with FACS Diva Software (BD Bioscience) and analyzed using FlowJo software.

In some experiments, isolated CD3₊ T cells from healthy donors were stained with the proliferation dye CFSE. Therefore, CD3+ T cells were resuspended in pre-warmed PBS to a final concentration of 2x10⁷/ml and stained with 1 µM CFSE for 20 minutes at 37 °C in the dark. Afterwards, cells were washed twice with X-VIVO-15/10% HSA and further stored at 37 °C until use in X-VIVO-15.

### Mixed leukocyte reaction

A mixed leukocyte reaction served as the basis for the experiments. For this purpose, CD3+ T cells from healthy donors, MS patients, PSO patients or SSC patients were co-cultured with allogeneic DC (TC:DC ratio of 20:1) in the absence or presence of GP120 (ActiTrexx GmbH / Polymun Scientific GmbH) (1 µg/ml). In further approaches, different modulators were added to these cultures to investigate possible synergistic effects on T cell proliferation. The following modulators were used so far (Table 2):

**Table 2:**

| **Modulator** | **Supplier** | **Concentration** |
|---|---|---|
| Tocilizumab (RoActemra^{®}), anti-human IL6R mAb | Roche | 30 ng/ml |
| Ixekizumab (Taltz^{®}), anti-human IL-17A mAb | Eli Lilly | 50 ng/ml |
| Adalimumab (Amgevita^{®}), anti-human TNF-alpha mAb | Amgen | 50 ng/ml |
| rh IFN-beta-1a | Miltenyi Biotec | 1 IU/ml |
| Nerandomilast, PDE4B inhibitor | Hycultec | 2 µM |
| Dimethyl fumarate | Merck | 2 µM |

### Measurement of CD3+ T cell Proliferation

T cell proliferation was analyzed using two methods equally suitable for evaluating the experiments:
a) Incorporation of ³H-Tdr:
   On day 4 of culture, ³H-Tdr was added to each well (37 kBq/well) and cells were cultured for an additional 16 h in an incubator at 37 °C and 5% CO₂. Pan CD3+ T cell proliferation was measured by ³H-Tdr incorporation using a liquid beta-scintillation counter.
b) Flow cytometric analysis of CFSE-labeled CD3+ T cells:
   Some experiments were performed using CFSE-labeled CD3+ T cells. On day 6 of culture, cells were harvested and stained with the viability dye Zombie NIR (Biolegend) and anti-human CD4 PE-Cy7 (RPA-T4) antibody (BD Pharmingen). Stained cells were measured on LSRII with FACS Diva Software (BD Bioscience) and analyzed using FlowJo software. The viability dye Zombie NIR enabled the distinction between live and dead cells, while CD4 staining differentiated CD4⁺ T cells from CD4⁻ T cells (equivalent to CD8⁺ T cells). The CFSE signal was assessed within the viable CD4⁺ and CD4⁻ T cell populations.
Non-proliferating cells exhibited a strong CFSE signal. With each cell division, the CFSE fluorescence intensity was progressively halved in the daughter cells. Thus, the gradual reduction of the CFSE signal served as an indicator of T cell proliferation.

### Preparation of activated Treg

Regulatory T cells from the blood of healthy donors (buffy coats or leukapheresis products) were isolated using immunomagnetic beads (anti-human CD25 microbeads, available from Miltenyi) and stimulated with GP120 as previously described¹⁻³. These stimulated Treg population (activated Treg) were used in the assays in a ratio of 4:1 (four T cells to one activated Treg cell) and cocultured with allogeneic DC in a ratio of 20:1 (20 T cells to one DC)⁴.

### References:

1. Protection from graft-versus-host disease by HIV-1 envelope protein gp120-mediated activation of human CD4+CD25+ regulatory T cells. Becker C, Taube C, Bopp T, Becker C, Michel K, Kubach J, Reuter S, Dehzad N, Neurath MF, Reifenberg K, Schneider FJ, Schmitt E, Jonuleit H. Blood. 2009 Aug 6;114(6):1263-9. doi: 10.1182/blood-2009-02-206730. Epub 2009 May 13.
2. Interferon-Beta Therapy of Multiple Sclerosis Patients Improves the Responsiveness of T Cells for Immune Suppression by Regulatory T Cells. Trinschek B, Luessi F, Gross CC, Wiendl H, Jonuleit H. Int J Mol Sci. 2015 Jul 17;16(7):16330-46. doi: 10.3390/ijms160716330.
3. Dimethyl Fumarate Therapy Significantly Improves the Responsiveness of T Cells in Multiple Sclerosis Patients for Immunoregulation by Regulatory T Cells. Schlöder J, Berges C, Luessi F, Jonuleit H. Int J Mol Sci. 2017 Jan 28;18(2):271. doi: 10.3390/ijms18020271.
4. Pro-inflammatory cytokines and prostaglandins induce maturation of potent immunostimulatory dendritic cells under fetal calf serum-free conditions. Jonuleit H, Kühn U, Müller G, Steinbrink K, Paragnik L, Schmitt E, Knop J, Enk AH. Eur J Immunol. 1997 Dec;27(12):3135-42. doi: 10.1002/eji.1830271209.

## Claims

1. An immunologically effective combination, comprising at least one immunomodulatory agent and a preparation of isolated CD4-activated regulatory T cells (activated Treg) for use in the prevention or treatment of an autoinflammatory disease or autoimmune disease.

2. The immunologically effective combination for the use according to claim 1, wherein said
a. immunomodulatory agent modulates, suppresses or modifies immune responses in a subject without specifically targeting immune tolerance mechanisms,
b. activated Treg promote and restore immune tolerance and thereby suppress autoinflammatory and autoimmune T effector cells.

3. The immunologically effective combination for the use according to claim 1 or claim 2, wherein said immunomodulatory agent is selected from the group consisting of
a. IL-6R inhibitors, IL-17 inhibitors, IL-23 inhibitors, TNF-alpha inhibitors, PDE4 inhibitors, interferon, fumarate immunomodulators, and/or
b. methotrexate, retinoids, IL-2, CD20 inhibitors, CD80/86 inhibitors, hydroxychloroquine, integrin blockers, sphingosine 1-phosphate receptor modulator, lymphocyte-depleting agents, glatiramer acetate, CD40 inhibitors, preferably CD40L blockers, pyrimidine synthesis inhibitors, B cell inhibitors.

4. The immunologically effective combination for the use according to claim 3, wherein said
a. IL-6R inhibitor is an anti-IL6R antibody,
b. IL-17 inhibitor is an anti-IL17RA antibody or an anti-IL17A antibody,
c. IL-23 inhibitor is an anti-IL23R antibody,
d. TNF-alpha inhibitor is an anti-TNF-alpha antibody, or a TNFR-IgG fusion protein,
e. PDE4 inhibitor is an PDE4B inhibitor,
f. interferon is interferon-beta-1a or interferon-beta-1b,
g. fumarate immunomodulator is dimethyl fumarate (DMF), diroximel fumarate or monomethyl fumarate.

5. The immunologically effective combination for the use according to claim 3, wherein said IL-6 inhibitor is Tocilizumab, or Sarilumab,
said IL-17 inhibitor is Ixekizumab, Secukinumab, Bimekizumab, or Brodalumab,
said IL-23 inhibitor is Risankizumab, Ustekinumab, or Guselkumab,
said TNF-alpha inhibitor is Adalimumab, Etanercept, or Infliximab,
said PDE4 inhibitor is Nerandomilast, Roflumilast, or Apremilast,
said interferon is interferon-beta-1a,
said fumarate immunomodulator is DMF.

6. The immunologically effective combination for the use according to claim 3, wherein said CD20 inhibitor is Rituximab, Ocrelizumab, Ublituximab, or Ofatumumab,
said CD80/86 inhibitor is Abatacept,
said integrin blocker is Vedolizumab,
said sphingosine 1-phosphate receptor modulator is Figolimod, Ozanimod, Ponesimod, or Siponimod,
said lymphocyte-depleting agent is Alemtuzumab,
said CD40L blocker is Frexalimab or Dazodalibep,
said pyrimidine synthesis inhibitor is Vidofludimus Calcium,
said IL-2 is Aldesleukin,
said B cell inhibitor is Belimumab.

7. The immunologically effective combination for the use according to any one of claims 1 to 6, wherein the activated Treg were produced by isolating regulatory T cells from blood of autologous or allogeneic donors and subsequent purification of said regulatory T cells.

8. The immunologically effective combination for the use according to claim 7, said regulatory T cells were isolated using anti-CD25 immunomagnetic beads and stimulated with anti-CD4 antibodies or functional fragments thereof, or HIV-1 glycoprotein 120 (GP120) or biologically active fragments thereof to produce activated Treg.

9. The immunologically effective combination for the use according to claim 7, wherein the anti-CD4 antibody used for stimulating regulatory T cells is Tregalizumab or Palixizumab.a

10. The immunologically effective combination for the use according to any one of claims 1 to 9, wherein the at least one immunomodulatory agent is provided in a first dosage form and the activated Treg are provided as a second dosage form, wherein a dosage regimen provides for the first dosage form to be administered to a subject concurrently with or prior to the second dosage form.

11. The immunologically effective combination for the use according to any one of claims 1 to 10, wherein the autoinflammatory disease is any one of psoriasis, rheumatoid arthritis, Sjögren's syndrome, diabetes type-1, Crohn's disease and ulcerative colitis, multiple sclerosis, psoriatic arthritis, systemic lupus erythematosus (SLE), uveitis, systemic sclerosis, neuro sarcoidosis.

12. The immunologically effective combination for the use according to claim 11, wherein for the treatment of
a. psoriasis, the immunomodulatory agent is any one of TNF-alpha inhibitor, IL-17 inhibitor, PDE4 inhibitor, interferon, or fumarate immunomodulator;
b. multiple sclerosis, the immunomodulatory agent is any one of fumarate immunomodulator, TNF-alpha inhibitor, IL-6R inhibitor, IL-17 inhibitor, PDE4 inhibitor, or interferon;
c. systemic sclerosis, the immunomodulatory agent is any one TNF-alpha inhibitor, IL-6R inhibitor, or PDE4 inhibitor;
d. rheumatoid arthritis, the immunomodulatory agent is any one TNF-alpha inhibitor, IL-6R inhibitor, or PDE4 inhibitor.
e. Sjögren's syndrome, the immunomodulatory agent is a PDE4 inhibitor,
f. diabetes type-1, the immunomodulatory agent is a PDE4 inhibitor,
g. Crohn's disease and ulcerative colitis, the immunomodulatory agent is any one of TNF-alpha inhibitor, or PDE4 inhibitor,
h. psoriatic arthritis, the immunomodulatory agent is any one of IL-17 inhibitor, TNF-alpha inhibitor, or PDE4 inhibitor,
i. systemic lupus erythematosus (SLE), the immunomodulatory agent is a PDE4 inhibitor,
j. uveitis, the immunomodulatory agent is any one of TNF-alpha inhibitor, or PDE4 inhibitor,
k. neuro sarcoidosis, the immunomodulatory agent is a TNF-alpha inhibitor.

13. A pharmaceutical composition, comprising an immunologically effective combination according to any one of claims 1 to 10 and a pharmaceutically acceptable carrier or diluent.

14. A pharmaceutical composition, comprising an immunologically effective combination according to any one of claims 1 to 10 and a pharmaceutically acceptable carrier or diluent for the use in the prevention or treatment of an autoimmune disease or autoinflammatory disease.

15. The pharmaceutical composition according to claim 13 or claim 14, wherein the immunomodulatory agent and the activated Treg in the immunologically effective combination are provided in a single dose form or in two distinct dose forms.

16. Use of an immunologically effective combination comprising at least one immunomodulatory agent and a preparation of isolated CD4-activated regulatory T cells (activated Treg) according to any one of claims 1 to 10 for the manufacturing of a medicament that promotes and restores immune tolerance in a human or animal subject.
